# EUROPEAN PATENT APPLICATION

(11) **EP 4 130 202 A1**
(43) Date of publication of application: **08.02.2023**
(21) Application number: 21776355.6
(22) Date of filing: 25.03.2021
(51) Int. Cl.: C10G 3/00, C10L 1/02

(54) **METHOD FOR PRODUCING BIODIESEL FUEL**

(30) Priority: 25.03.2020 JP 2020055203
(71) Applicant: Biofuel Technology Research Co., Ltd., Hiroshima-shi, Hiroshima 731-0103 (JP); Marubeni Corporation, Chiyoda-ku, Tokyo 100-8088 (JP)
(72) Inventor: KAJIMA Hiroshi, Hiroshima-shi, Hiroshima 731-0103 (JP)
(74) Representative: Lahrtz, Fritz
(86) International application number: PCT/JP2021/012757
(87) International publication number: WO 2021/193887

(57) **Abstract**

Provided are a first separation step of mixing a raw material containing at least one of a free fatty acid and a fatty acid glycerin ester with an inorganic acid or enzyme to separate a first oil component and a first glycerin liquid; and a catalytic cracking step of bringing an oil component or a fatty acid alkyl ester into contact with a catalyst to obtain a hydrocarbon, wherein (a) the above first oil component is used in the above catalytic cracking step, or (b) an esterification step of reacting the first oil component obtained in the first separation step with a monohydric alcohol to obtain a fatty acid alkyl ester by using a method other than an alkali catalyst method is further provided, and the obtained fatty acid alkyl ester is used as a raw material in the above catalytic cracking step. According to the present invention, there is provided a method for producing a biodiesel fuel whose main components are hydrocarbons, that is, a novel production method that can use a raw material containing at least one of a free fatty acid and a fatty acid glycerin ester.

## Description

### [Technical Field]

The present invention relates to a method for producing a biodiesel fuel and more particularly to a method that can use wastes such as oil/fat-containing wastes, waste cooking oil, and waste glycerin as raw materials.

### [Background Art]

In recent years, from the viewpoint of preventing global warming, the development of fuels that replace conventional fossil fuels has been promoted, including those reducing the generation of carbon dioxide and leading to the recycling of resources. As one of them, biodiesel fuels whose raw materials are plant oil, waste cooking oil, and the like are attracting attention. The mainstream method of synthesizing a biodiesel fuel is a method in which a fatty acid alkyl ester is synthesized by a transesterification reaction using animal/plant oil/fat and a monohydric alcohol as the raw materials and an alkaline substance such as potassium hydroxide as the catalyst (e.g., Non-Patent Document 1). In recent years, however, a biodiesel fuel whose main components are hydrocarbons has been attracting attention as a so-called "next-generation biodiesel fuel."

For such a next-generation biodiesel fuel, Patent Document 1 proposes a method of producing hydrocarbons by a catalytic cracking method using oil/fat such as plant oil or waste cooking oil as a raw material. According to such a method, among fatty acid glycerin esters, hydrocarbons derived from hydrocarbon groups of fatty acids, propane derived from glycerin moieties, and carbon dioxide and carbon monoxide derived from ester groups are generated.

### [Prior Art Documents]

[Patent Document 1] JP5234456B

### [Non-Patent Documents]

[Non-Patent Document 1] Journal of the Japan Institute of Marine Engineering, 2012, Vol. 47, No. 1, pp. 45-50

### [Summary of the Invention]

### [Problems to be solved by the Invention]

In the production of a so-called "next-generation biodiesel fuel" whose main components are hydrocarbons, raw materials that can be used are limited. For example, it is difficult to use, as the raw materials, fatty acid glycerin ester-containing wastes such as waste glycerin, which is produced as a by-product of the production of fatty acid alkyl ester or the like, and free fatty acid-containing wastes such as a high acid value oil, which has an extremely high acid value, among waste cooking oils. In recent years, the treatment of such wastes has become an urgent issue, and the development of a method that can utilize these wastes as the raw materials is desired also in the production of next-generation biodiesel fuel.

The present invention has been made in view of the above problems, and an object of the present invention is to provide a method for producing a biodiesel fuel whose main components are hydrocarbons, that is, a novel production method that can use a raw material containing at least one of a free fatty acid and a fatty acid glycerin ester.

### [Means for solving the Problems]

As a result of intensive studies to solve the above problems, the present inventor has found that a biodiesel fuel whose main components are hydrocarbons can be efficiently produced through adding an inorganic acid or enzyme to a raw material containing at least one of glycerin and a fatty acid glycerin ester to separate them and using the obtained oil component, or using a fatty acid alkyl ester obtained by further esterifying the oil component, to subject the above oil component or fatty acid alkyl ester to a catalytic cracking method. The present inventor has thus accomplished the present invention.

Specifically, the present invention is as follows.

(1) A method for producing a biodiesel fuel, comprising:
   a first separation step of mixing a raw material containing at least one of a free fatty acid and a fatty acid glycerin ester with an inorganic acid or enzyme to separate a first oil component and a first glycerin liquid;
   an esterification step of reacting the first oil component with a monohydric alcohol to obtain a fatty acid alkyl ester by using a method other than an alkali catalyst method, the method being at least one method selected from the group consisting of an acid catalyst method, an acid-alkali catalyst method, a biocatalyst method, an ion-exchange resin method, a supercritical method, a subcritical method, and a solid catalyst method; and
   a catalytic cracking step of bringing the fatty acid alkyl ester into contact with a catalyst to obtain a hydrocarbon.
(2) The method described in (1), comprising:
   a neutralization step of neutralizing, with an alkaline substance, the first glycerin liquid obtained by using the inorganic acid in the first separation step; and
   a second separation step of separating a second oil component and a precipitated inorganic salt from the neutralized glycerin liquid,
   wherein the esterification step includes reacting the second oil component together with the first oil component.
(3) The method described in (1) or (2), comprising:
   a neutralization step of neutralizing, with an alkaline substance, the first glycerin liquid obtained by using the inorganic acid in the first separation step;
   a second separation step of separating a second oil component and a precipitated inorganic salt from the neutralized glycerin liquid; and
   an alcohol separation step of separating a monohydric alcohol from the glycerin liquid from which the second oil component and the inorganic salt are separated,
   wherein the monohydric alcohol separated in the alcohol separation step is used as the monohydric alcohol in the esterification step.
(4) A method for producing a biodiesel fuel, comprising:
   a first separation step of mixing a raw material containing at least one of a free fatty acid and a fatty acid glycerin ester with an inorganic acid or enzyme to separate a first oil component and a first glycerin liquid; and
   a catalytic cracking step of bringing the first oil component into contact with a catalyst to obtain a hydrocarbon.
(5) The method described in (4), comprising:
   a neutralization step of neutralizing, with an alkaline substance, the first glycerin liquid obtained by using the inorganic acid in the first separation step; and
   a second separation step of separating a second oil component and a precipitated inorganic salt from the neutralized glycerin liquid,
   wherein the catalytic cracking step includes bringing the second oil component into contact with the catalyst together with the first oil component.
(6) The method described in any one of (1) to (5), wherein the raw material in the first separation step contains at least one of a high acid value oil having an acid value of 10 mgKOH/g or more and waste glycerin produced as a by-product in a process of producing a fatty acid alkyl ester.
(7) The method described in any one of (1) to (6), wherein the inorganic acid is used in the first separation step, and the inorganic acid is concentrated sulfuric acid.
(8) The method described in any one of (1) to (7), wherein the inorganic acid is used in the first separation step, and a mixed liquid of the raw material and the inorganic acid has a pH of 3 or less.
(9) The method described in any one of (1) to (8), wherein the catalyst in the catalytic cracking step contains magnesium oxide.
(10) The method described in any one of (1) to (9), wherein the catalytic cracking step includes performing a reaction in a rotating drum that is arranged in a state in which its axis of rotation is inclined with respect to a horizontal plane.
(11) The method described in any one of (1) to (10), wherein a reaction temperature in the catalytic cracking step is 200°C to 600°C.
(12) The method described in (11), wherein the reaction temperature is adjusted by electromagnetic induction heating.

### [Effect of the Invention]

According to the present invention, a raw material containing at least one of a free fatty acid and a fatty acid glycerin ester can be used in the method for producing a biodiesel fuel whose main components are hydrocarbons, and free fatty acid-containing wastes and fatty acid glycerin ester-containing wastes, for example, can be effectively recycled.

### [Brief Description of Drawings]

FIG. 1 is a diagram illustrating a flow for obtaining a first oil component in a first embodiment of the present invention.
FIG. 2 is a diagram illustrating a flow for obtaining a fatty acid alkyl ester in a second embodiment of the present invention.
FIG. 3 is a diagram illustrating a flow of a catalytic cracking step in an embodiment of the present invention.

### [Embodiments for Carrying out the Invention]

Hereinafter, embodiments of the present invention will be described.

The method for producing a biodiesel fuel according to an embodiment of the present invention includes: a first separation step of mixing a raw material containing at least one of a free fatty acid and a fatty acid glycerin ester with an inorganic acid or enzyme to separate a first oil component and a first glycerin liquid; and a catalytic cracking step of bringing a raw material containing an oil component or a fatty acid alkyl ester into contact with a catalyst to obtain a hydrocarbon.

In this method:
(a) the above first oil component may be used in the above catalytic cracking step (referred to as a "first embodiment," hereinafter); or
(b) the method may further include an esterification step of reacting the first oil component obtained in the first separation step with a monohydric alcohol to obtain a fatty acid alkyl ester by using a method other than an alkali catalyst method, the method being at least one method selected from the group consisting of an acid catalyst method, an acid-alkali catalyst method, a biocatalyst method, an ion-exchange resin method, a supercritical method, a subcritical method, and a solid catalyst method, and the obtained fatty acid alkyl ester may be used as a raw material in the above catalytic cracking step (referred to as a "second embodiment," hereinafter).

### <First Embodiment>

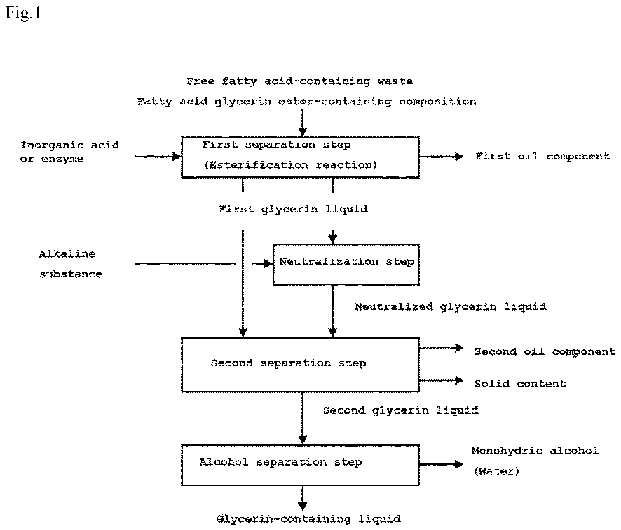

FIG. 1 is a diagram illustrating a flow for obtaining the first oil component in the first embodiment. FIG. 1 is illustrated such that, in addition to the first separation step for obtaining the first oil component (first separation step), a neutralization step, a second separation step, and an alcohol separation step may be carried out as optional steps after the first separation step.

As will be described later, wastes containing a free fatty acid and/or a fatty acid glycerin ester contain other components. Therefore, if it is attempted to apply the wastes directly to the production reaction for biodiesel fuel (catalytic cracking method), problems may arise in that the catalytic cracking reaction is inhibited or a large amount of impurities remains in the product. Fortunately, however, according to the present embodiment, the content of impurities such as glycerin and inorganic salts is reduced by the (first) separation step, and the wastes can therefore be suitably applied to the catalytic cracking method. In addition, there are various methods for producing a fatty acid alkyl ester, a free fatty acid, etc., and accordingly there is a problem in that the quality of wastes containing a free fatty acid and/or a fatty acid glycerin ester is not stable, but according to the present embodiment, it is possible to produce the biodiesel fuel (whose main components are hydrocarbons) with stabilized quality.

Furthermore, in the present embodiment, by performing the (first) separation step using an inorganic acid or enzyme, various raw materials including not only free fatty acid-containing wastes but also fatty acid glycerin ester-containing wastes can be treated by the present method.

Conventionally, methods for producing hydrocarbons by the catalytic cracking method have been proposed for fatty acid glycerin ester-containing wastes, including waste cooking oil (e.g., Patent Document 1 as described previously). When the fatty acid glycerin ester is directly subjected to the catalytic cracking method, lower hydrocarbons (hydrocarbons whose carbon number is 4 or less) such as propane derived from the glycerin moieties are generated, but in order to recover such lower hydrocarbons, investment for facilities is required and the cost is thus high. On the other hand, when the recovery facilities for the lower hydrocarbons are not provided, there is a problem in that the glycerin moieties cannot be recycled.

In contrast, according to the present embodiment, a transesterification reaction occurs in the (first) separation step to produce glycerin. Such glycerin can be recovered separately and applied to various uses. Thus, according to the present embodiment, the glycerin moieties of the fatty acid glycerin ester can be effectively recycled even when the recovery facilities for the lower hydrocarbons are not provided.

### (1) Raw Material

The raw material used in the present embodiment is not particularly limited, provided that it contains at least one of a free fatty acid and a fatty acid glycerin ester.

Examples of the raw material containing a free fatty acid include wastes that contain a free fatty acid. Examples of the raw material containing a fatty acid glycerin ester include wastes that contain a fatty acid glycerin ester.

The free fatty acid-containing waste and the fatty acid glycerin ester-containing waste will be described below in some detail.

### (1-1) Free Fatty Acid-containing Waste

Examples of the free fatty acid-containing waste that can be used in the present embodiment include waste glycerin produced as a by-product in a process of producing a biodiesel fuel, sweet water, and washing wastewater for a fatty acid alkyl ester.

Here, the wastewater for a fatty acid alkyl esters is wastewater generated when a reaction product is washed in the process of producing a fatty acid alkyl ester such as biodiesel fuel, and may contain an unreacted free fatty acid and its salt in addition to water and may further contain glycerin produced as a by-product in the production reaction of a fatty acid alkyl ester, an unreacted monohydric alcohol, etc.

The sweet water is a by-product in the case of saponifying oil/fat (performing alkaline hydrolysis of oil/fat) to produce a fatty acid salt (e.g., in the process of producing soap or the like) and may contain glycerin, water, alkali, and the like.

The waste glycerin produced as a by-product in the process of producing a biodiesel fuel will then be described in some detail.

The fatty acid alkyl ester to be a biodiesel fuel can be obtained through adding a monohydric alcohol such as methanol and an alkali catalyst such as potassium hydroxide to raw oil/fat such as plant oil and performing a transesterification reaction.

Examples of the raw oil/fat to be used for a biodiesel fuel include plant oils such as rapeseed oil, palm oil, olive oil, sunflower oil, soybean oil, rice oil, and cannabis oil; animal oils such as fish oil, pork fat, and beef and pork fats; and waste cooking oil such as tempura oil.

Examples of the monohydric alcohol for use include methanol, ethanol, 1-propanol, and ethylhexanol, among which methanol and ethanol are preferred, and methanol is particularly preferred.

Examples of the alkali catalyst for use include potassium hydroxide, sodium hydroxide, and calcium oxide, but potassium hydroxide is preferred from the viewpoints of precipitation property, ease of reuse, and the like of the inorganic salt which is separated and recovered in the present embodiment.

In the above transesterification reaction, the fatty acid glycerin ester contained in the raw oil/fat reacts with the monohydric alcohol to produce a fatty acid alkyl ester and glycerin. The obtained reaction liquid is separated into two liquid phases of a fatty acid alkyl ester phase and a waste glycerin phase, and in the production of a biodiesel fuel, the obtained fatty acid alkyl ester phase may be recovered and subjected to washing and other necessary treatment to obtain a biodiesel fuel.

On the other hand, the waste glycerin phase contains not only glycerin at a high concentration but also an unreacted monohydric alcohol, unreacted oil/fat, a fatty acid and its salt, an alkali catalyst, and foreign substances derived from the raw oil/fat. The waste glycerin may be liquid waste glycerin or solid waste glycerin, but from the viewpoints of workability, handling, and the like, liquid waste glycerin is preferred.

The content of glycerin, a monohydric alcohol, oil/fat, and a free fatty acid and its salt in the waste glycerin is not particularly limited, but usually, the content of glycerin is 25 mass% or more and 65 mass% or less, the content of a monohydric alcohol is 2 mass% or more and 20 mass% or less, and the total content of oil/fat and a free fatty acid and its salt is 30 mass% or more and 50 mass% or less in many cases with respect to the waste glycerin as a whole. From the viewpoint of enabling the method for producing a biodiesel fuel according to the present embodiment to be more stably carried out, the content of glycerin may be 30 mass% or more and 65 mass% or less, the content of a monohydric alcohol may be 3 mass% or more and 15 mass% or less, and the total content of oil/fat and a fatty acid and its salt may be 25 mass% or more and 55 mass% or less with respect to the waste glycerin as a whole.

The pH of waste glycerin is often 9 or more because it contains a large amount of alkali catalyst, and in the present embodiment, the pH may be 9 to 13.

When an inorganic acid is used in the first separation step, from the viewpoint of allowing the acid-catalyzed esterification reaction to readily progress between the unreacted oil/fat and the monohydric alcohol contained in the waste glycerin, the water content in the waste glycerin is preferably 5 mass% or less and particularly preferably 3 mass% or less. The water content in the waste glycerin can be appropriately adjusted by heating, pressure reduction, use of a desiccant or the like, permeation through purified glycerin, etc.

In the present embodiment, from the viewpoint of availability in the first separation step, which will be described later, it is particularly preferred to use the waste glycerin produced as a by-product in the process of producing a biodiesel fuel, among the free fatty acid-containing wastes described above.

### (1-2) Fatty Acid Glycerin Ester-containing Composition

In the present embodiment, a composition that contains a fatty acid glycerin ester can also be used as a raw material. In the present embodiment, the composition containing a fatty acid glycerin ester may be used to cause an esterification reaction or the like in the first separation step because it is performed in the (first) separation step using an inorganic acid or enzyme. This allows the free fatty acid to be recovered, and the glycerin can be separated.

Examples of the composition containing a fatty acid glycerin ester include oil/fat whose main component is a fatty acid glycerin ester of waste cooking oil, animal oil (such as beef fat, pork oil, bird oil, butter, waste milk, fish oil, or cod-liver oil), plant oil (such as rapeseed oil, palm oil, olive oil, sunflower oil, soybean oil, rice oil, cannabis oil, or margarine), or high acid value oil (such as grease trap oil, sewage oil, gutter oil, waste liquid treatment recycled oil, mayonnaise, or dressing); a composition whose main component is a fatty acid salt of an oil cake, soap, or the like; and a waste liquid produced as a by-product in the process of producing a free fatty acid.

In the present specification, the "main component" means a component having the highest content in the composition (when the component having the highest content is water, a component having the second highest content). The content of the main component is preferably 40 mass% or more and more preferably 50 mass% or more.

Here, the high acid value oil refers to oil/fat having an acid value of 10 mgKOH/g or more, and examples thereof include a free fatty acid in addition to a fatty acid glycerin ester that is the main component of oil/fat. The acid value may be 20 mgKOH/g or more in an embodiment or 50 mgKOH/g or more in another embodiment. The upper limit of the acid value is usually 200 mgKOH/g or less.

The oil cake is a by-product separated from oil/fat (raw oil) in a deacidification step in the purification of plant oil/fat and contains a fatty acid salt, a fatty acid glycerin ester, alkali, water, and the like.

The waste liquid produced as a by-product in the step of producing a free fatty acid refers to waste produced as a by-product when hydrolyzing animal/plant oil/fat to produce a free fatty acid. Examples of the method for producing a free fatty acid by hydrolysis include a high-temperature and high-pressure decomposition method and an enzymatic decomposition method. The waste liquid produced as a by-product in such a production step contains glycerin generated by the hydrolysis, partially hydrolyzed oil/fat, and the like in addition to unreacted oil/fat.

### (2) First Separation Step

The separation step (this step may be referred to as a "first separation step," hereinafter, in comparison with a second separation step, which will be described later) is a step of mixing the raw material, which contains at least one of a free fatty acid and a fatty acid glycerin ester, with an inorganic acid or enzyme to phase-separate a first oil component and a first glycerin liquid.

The oil component separated in this step may contain a free fatty acid and a fatty acid glycerin ester in addition to a fatty acid alkyl ester.

When using a raw material that contains a free fatty acid, a fatty acid alkyl ester is generated by an esterification reaction with an unreacted monohydric alcohol contained in the waste glycerin using the inorganic acid or enzyme as a catalyst.

When the fatty acid exists as a salt, it is preferred to use an inorganic acid in this step because the salt of the fatty acid is converted to a free fatty acid by the inorganic acid and is easily separated from the glycerin.

When using a fatty acid glycerin ester-containing composition as the raw material, a fatty acid alkyl ester and glycerin are generated by a transesterification reaction with the monohydric alcohol. Such glycerin can be recovered separately as will be described later, and therefore the glycerin moieties of the fatty acid glycerin ester can also be effectively recycled without providing recovery facilities for lower hydrocarbons.

The monohydric alcohol in the above transesterification reaction can be added separately, and for example, the monohydric alcohol recovered in the alcohol separation step, which will be described later, can be used. Additionally or alternatively, waste glycerin may be treated at the same time as the fatty acid glycerin ester-containing composition thereby to use an unreacted monohydric alcohol contained in the waste glycerin.

When performing the first separation step under the presence of a monohydric alcohol, this step can also be referred to as an esterification step. In comparison with the second esterification reaction, which will be described later, the first separation step may be referred to as a "first esterification step."

Even when the monohydric alcohol is not contained, the fatty acid glycerin ester generates a free fatty acid, glycerin, and a partially hydrolyzed fatty acid glycerin ester (monoglyceride, diglyceride) under the presence of an acid in the first separation step. Additionally or alternatively, when the raw material contains a fatty acid salt, the fatty acid salt is converted by the acid into a free fatty acid, which may readily be separated from the glycerin.

Therefore, the present embodiment can be preferably applied even when the raw material contains no monohydric alcohol.

In the present embodiment, various raw materials can be treated at the same time because the first separation step is performed under the presence of an inorganic acid or enzyme. Moreover, by performing the first separation step, the waste such as waste glycerin, waste cooking oil, or high acid value oil, which contains a free fatty acid and/or a fatty acid glycerin ester, can be effectively utilized, and this can therefore contribute to reduction of the environmental load.

Among these, the high acid value oil has a high acid value of 10 mgKOH/g or more and may therefore be difficult to use as a raw material for the transesterification reaction using the previously described alkali catalyst. Fortunately, however, the high acid value oil can also be preferably used as a raw material in the first separation step, which should be also referred to as an esterification reaction, and the high acid value oil is particularly preferred as a raw material when using an inorganic acid.

When using waste glycerin, a fatty acid glycerin ester-containing composition, and the like as the raw materials, the fatty acid alkyl ester and free fatty acid generated in the first separation step migrate to an oil phase composed of the first oil component and can therefore be separated from the first glycerin liquid.

The water content in the raw material which can be used in the first separation step is preferably 10 mass% or less and preferably 5 mass% or less. By using a raw material having a low water content (e.g., waste glycerin or the like having a low water content), it becomes easy to reduce the water content in the reaction liquid, which will be described later. The water content in the raw material can be appropriately adjusted by heating, pressure reduction, use of a desiccant or the like, permeation through purified glycerin, etc.

When an inorganic acid is used in the first separation step, examples of the inorganic acid include concentrated sulfuric acid, phosphoric acid, concentrated nitric acid, and hydrogen chloride, but concentrated sulfuric acid and phosphoric acid having a low water content are preferred, and concentrated sulfuric acid is particularly preferred.

In the first separation step, the pH of the mixed liquid (reaction liquid) of the above raw material and the above inorganic acid is preferably 3 or less and particularly preferably 1 or less. The pH of the reaction liquid can be adjusted by the additive amount of the above inorganic acid.

The water content in the reaction liquid is preferably 10 mass% or less and particularly preferably 0.5 mass% or less. The water content in the reaction liquid can be appropriately adjusted by adjustment of the water content and charged amount of each raw material, use of a desiccant for the reaction liquid, etc.

By setting the pH and water content of the reaction liquid within the above ranges, the efficiency of the acid-catalyzed esterification reaction can be increased, and the first oil component and the first glycerin liquid (including the acidic glycerin phase and the inorganic salt) can be well separated.

The temperature of the reaction liquid in the first separation step can be set to 30°C to 64°C in an embodiment or 50°C to 60°C in another embodiment. The reaction time can be set to 0.5 to 12 hours in an embodiment, 4 to 12 hours in another embodiment, or 8 to 12 hours in still another embodiment. During this period, it is preferred to stir the reaction liquid.

After the above reaction (or stirring) is completed, the reaction liquid may be placed statically for 0.2 to 12 hours thereby to separate the first oil component, which contains a fatty acid alkyl ester, unreacted oil/fat, and the like, and the first glycerin liquid, which contains an acidic glycerin phase and/or an inorganic salt.

On the other hand, when an enzyme is used in the first separation step, the enzyme is not particularly limited, provided that it can catalyze the transesterification reaction. For example, lipase, phospholipase, acyltransferase, etc. can be used. Among these, lipase is particularly preferred. The enzyme is preferably immobilized on a carrier.

The pH, reaction temperature, reaction time, etc. of the reaction liquid can be adjusted as appropriate in accordance with the enzyme to be used. After the reaction, the reaction liquid may be placed statically for 0.2 to 12 hours thereby to separate the first oil component, which contains a fatty acid alkyl ester, unreacted fat/oil, etc., and the first glycerin liquid, which contains glycerin and the enzyme.

The first oil component (such as a fatty acid alkyl ester or a free fatty acid) obtained by recovering the oil phase can be used as a raw material in the catalytic cracking step, which will be described later.

When an inorganic acid is used in the first separation step, the pH of the obtained first oil component tends to be acidic due to the acid-catalyzed esterification reaction, and it is therefore preferred to adjust the pH so that it becomes neutral. Specifically, it is preferred to adjust the pH to 4.0 to 7.5 in an embodiment, 4.5 to 7.0 in another embodiment, or 5.0 to 6.5 in still another embodiment. By adjusting the pH of the oil component to fall within such a range, production of a biodiesel fuel whose main components are hydrocarbons can be stably realized in the catalytic cracking step, which will be described later.

An alkaline substance may be used to adjust the pH. Examples of the alkaline substance for use include hydroxides such as potassium hydroxide and sodium hydroxide. In the present embodiment, various substances can be used as the alkaline substance because the oil component can also be recovered by separation after the pH adjustment.

For example, an alkaline substance that contains a free fatty acid can be used as the above alkaline substance. Examples thereof include a by-product of an alkali-catalyzed transesterification reaction of fat/oil, such as the above waste glycerin; and a composition whose main component is a fatty acid salt, such as an oil cake or alkaline soap. These can not only neutralize an acidic oil component but also increase the yield of a free fatty acid and are thus preferred from such viewpoints.

The first oil component obtained as described above can be used as a raw material in the catalytic cracking step, which will be described later. On the other hand, the first glycerin liquid may be subjected to a neutralization step and a second separation step, which will be described later, to further recover an oil component, and may be subjected to an alcohol separation step to recover a monohydric alcohol.

### (3) Neutralization Step

The neutralization step is a step of neutralizing the first glycerin liquid, which is obtained when an inorganic acid is used in the first separation step, with an alkaline substance. When an enzyme is used in the first separation step as illustrated in FIG. 1, the neutralization step can be omitted.

Examples of such an alkaline substance for use include hydroxides such as potassium hydroxide and sodium hydroxide. In the present embodiment, various substances can be used as the alkaline substance because the second separation step is performed after the neutralization step.

For example, a substance that contains glycerin can be used as the above alkaline substance. Examples of such a glycerin-containing alkaline substance include by-products obtained by the alkali-catalyzed transesterification reaction of oil/fat, such as the above waste glycerin. These can not only neutralize acidic glycerin but also increase the yield of glycerin, and therefore the use of a glycerin-containing alkaline substance is preferred also from such a viewpoint. The glycerin-containing alkaline substance may contain a fatty acid salt and/or a fatty acid glycerin ester.

The glycerin content in the above glycerin-containing alkaline substance is preferably 25 mass% or more and particularly preferably 50 mass% or more. The upper limit is not particularly limited, but may be, for example, 99 mass% or less in an embodiment or 90 mass% or less in another embodiment.

The pH of the above glycerin-containing alkaline substance is preferably 9 or more and particularly preferably 9 to 13.

Additionally or alternatively, a composition whose main component is a fatty acid salt may be used as the above alkaline substance. Examples of the alkaline substance whose main component is a fatty acid salt include an oil cake and alkaline soap.

In the above neutralization step, the neutralization is preferably performed so that the pH of the glycerin liquid becomes 4.0 to 7.5 in an embodiment, 4.5 to 7.0 in another embodiment, or 5.0 to 6.5 in still another embodiment. By neutralizing the glycerin liquid so that the pH is within such a range, the oil component can be readily separated and the inorganic salt can be readily precipitated in the subsequent second separation step. The pH of the glycerin liquid can be appropriately adjusted by controlling the additive amount of the above alkaline substance.

The water content in the glycerin liquid obtained in the neutralization step is preferably 10 mass% or less and particularly preferably 3 mass% or less. In the neutralization step, a problem such as reaction inhibition due to water does not occur, but from the viewpoint of sufficiently precipitating the inorganic salt in the subsequent second separation step to improve the separation efficiency, it is preferred to specify the upper limit of the water content as described above. The lower limit of the water content is not particularly limited, but may be, for example, 0.5 mass% or more.

In the neutralization step, it is preferred to add the above alkaline substance while stirring the acidic glycerin liquid so that the liquid property shifts from acidic to near neutral. As previously described, a substance that contains a fatty acid salt may be used as the alkaline substance used for neutralization, in which case the addition order as described above allows the fatty acid salt to be converted into a free fatty acid by the acid. The free fatty acid migrates from the glycerin liquid to the oil phase which is phase-separated from the glycerin liquid, and is less likely to redissolve into the glycerin liquid even when the pH of the glycerin liquid becomes high. This allows the separation in the subsequent second separation step to be easier. The fatty acid salt is contained not only in the above-described substance whose main component is a fatty acid salt, but also in a by-product of an alkali-catalyzed transesterification reaction or alkali hydrolysis of oil/fat.

The above alkaline substance neutralizes the first glycerin liquid obtained in the first separation step. The neutralized glycerin liquid may be subjected to the subsequent second separation step.

### (4) Second Separation Step

The second separation step is a step of separating a second oil component and a solid content from the glycerin liquid obtained in the above step. The glycerin liquid obtained by separating the second oil component and the solid content in this step may be referred to as a second glycerin liquid.

Here, when an inorganic acid is used in the first separation step, as illustrated in FIG. 1, the above-described neutralization step is performed, and the neutralized glycerin is subjected to the second separation step.

In this case, the second oil component separated contains not only oil/fat and a fatty acid that have not been separated even in the first separation step and remain in the first glycerin liquid, but also oil/fat, a free fatty acid, and the like that are derived from the alkaline substance added in the neutralization step.

The solid content separated in the second separation step is an inorganic salt precipitated. The inorganic salt is a salt of an inorganic acid (such as concentrated sulfuric acid) added in the first separation step and an alkali (such as potassium or sodium) and is preferably potassium sulfate. The above alkali is contained in the raw material (such as waste glycerin) charged in the first separation step and/or the alkaline substance added in the neutralization step, and the inorganic salt is precipitated in the first separation step and/or the neutralization step.

On the other hand, when an enzyme is used in the first separation step, as illustrated in FIG. 1, the first glycerin liquid obtained in the first separation step is directly subjected to the second separation step.

In this case, the first glycerin liquid contains oil/fat, a fatty acid, etc. that have not been separated even in the first separation step, and these are separated as the second oil in this step. When an immobilized enzyme is used, it is separated as a solid content in the second separation step.

The second glycerin liquid separated in this step contains not only glycerin but also a monohydric alcohol derived from the waste glycerin and may contain water and the like. The oil component and the inorganic salt have low solubility in the second glycerin liquid and therefore are separated from the second glycerin liquid.

In the second separation step, after the glycerin liquid (the neutralized glycerin liquid when an inorganic acid is used; the first glycerin liquid when an enzyme is used) is placed statically for about 3 to 12 hours, the upper liquid (oil component) and the lower liquid (second glycerin liquid) are separately recovered, and the second glycerin liquid can be obtained as the lower liquid, but it is preferred to increase the separation speed by centrifugal separation or the like. In such centrifugal separation, a three-phase separation type centrifugal separator may be preferably used, which can separate a light liquid (i.e., the oil component), a heavy liquid (i.e., the second glycerin liquid), and a solid substance. When an organic acid is used in the first separation step, a large amount of inorganic salt is precipitated, and therefore it is also preferred to preliminarily separate a certain amount of inorganic salt using a centrifugal separator capable of solid-liquid separation, such as a decanter type centrifugal separator, and then further separate the liquid phase portion using a three-phase separation type centrifugal separator.

The second oil component obtained in the second separation step may be combined, for example, with the first oil component separated in the first separation step and subjected to the catalytic cracking step, which will be described later. In this case, it is preferred to adjust the pH of the oil component, and the specific method is as previously described for the first oil component.

Additionally or alternatively, the second oil component may be combined with the first oil component and subjected to a further esterification reaction (second esterification step described later) and can thereby be used to generate a fatty acid alkyl ester. Such a method will be described in detail in the second embodiment.

When an inorganic salt is recovered in this step, the inorganic salt can be used as a raw material for inorganic fertilizers, etc., for example, through a washing process and the like. On the other hand, when an immobilized enzyme is recovered in this step, the immobilized enzyme can be reused in the first separation step.

The second glycerin liquid can be used as a stripping agent for asphalt, a release agent for cement, a denitrifying agent or nitrification accelerator (organic carbon source for biological nitrification and denitrification treatment), etc., and can also be used for applications such as cosmetics by further performing purification.

When using a raw material that contains a monohydric alcohol (e.g., waste glycerin or the like which is produced as a by-product in the production step for biodiesel fuel (fatty acid alkyl ester)) in the first separation step, the second glycerin liquid may contain a monohydric alcohol. Such a monohydric alcohol can be used as a raw material for a (second) esterification step in the second embodiment, and it may therefore be further subjected to an alcohol separation step to recover the monohydric alcohol.

### (5) Alcohol Separation Step

The alcohol separation step is a step of separating a monohydric alcohol from the second glycerin liquid obtained in the second separation step.

The above second glycerin liquid may contain a monohydric alcohol that is derived from the waste glycerin and remains in the first separation step (esterification reaction). Such a monohydric alcohol can be used as a raw material for the (second) esterification step, which will be described later.

The alcohol separation step can employ a reduced-pressure distillation method, a gas-liquid contact method, a membrane separation method, or other similar method.

The reduced-pressure distillation method is a method in which the glycerin liquid is heated (e.g., to about 60°C) to vaporize the monohydric alcohol or the like, and then the pressure is reduced to separate the monohydric alcohol or the like. The separated monohydric alcohol or the like can be recovered by cooling.

The gas-liquid contact method is a method in which the glycerin liquid is brought into contact with the gas phase as fine droplets, and the monohydric alcohol having a low boiling point is made to migrate to the gas phase for separation. Specifically, a spray-drying method or the like can be preferably employed.

The membrane separation method is a method using a membrane that allows a monohydric alcohol to preferentially pass through.

The second glycerin liquid may further contain water. Such water may remain in the glycerin liquid, but can also be removed because it migrates to the gas phase together with the monohydric alcohol, for example, in the reduced-pressure distillation method, the gas-liquid contact method, or the like.

Additionally or alternatively, before or after the alcohol separation step of separating the above monohydric alcohol, further purification treatment may be performed using an ion-exchange method, activated white clay, diatomaceous earth, carbon, zeolite, or the like.

The separated monohydric alcohol can be reused as a raw material for the transesterification reaction without any modification or after being purified by re-distillation or the like as necessary.

In the glycerin liquid obtained in this step, the purity of glycerin is preferably 85 mass% or more, further preferably 90 mass% or more, especially preferably 97% or more, and particularly preferably 99 mass% or more. In the present embodiment, although the glycerin-containing waste is a raw material, even such a simple method allows highly pure glycerin to be obtained, such as within the above numerical range, through the above-described separation steps and alcohol separation step. The purity of glycerin refers to a value measured by gas chromatography.

According to the method described above, the first oil component and the second oil component can be separated from the raw material containing at least one of a free fatty acid and a fatty acid glycerin ester, and these can be used as raw materials for the catalytic cracking step, which will be described later.

### <Second Embodiment>

While the steps of the second embodiment partially overlap with those of the above-described first embodiment, the first oil component obtained in the (first) separation process is used as a raw material to produce a fatty acid alkyl ester, and the fatty acid alkyl ester is used as a raw material in the catalytic cracking step, which will be described later.

In the present embodiment, the second oil component obtained in the above second separation step may be used in addition to the above first oil component. Additionally or alternatively, the monohydric alcohol recovered by the above alcohol separation step may be used as a raw material.

In the above-described first and second separation steps, the first and second oil components are respectively recovered from the separated oil phase. In the above-described alcohol separation step, the separated monohydric alcohol is recovered. It is conceivable that these are circulated and supplied as raw materials in the production of a fatty acid alkyl ester by the alkali catalyst method, but the purity is not necessarily high; therefore, if it is attempted to use them as raw materials without any modification, it may be difficult to efficiently produce the fatty acid alkyl ester. Moreover, the first and/or second oil components contain oil/fat having a high acid value, such as a free fatty acid, and in particular, the first oil component when using an inorganic acid in the first separation step is an acidic oil component because it is separated in the first separation step (first esterification step) which can be said as an esterification reaction using an acid catalyst. Thus, it becomes more difficult to use the first and second oil components without any modification as the raw materials for producing a fatty acid alkyl ester using an alkali catalyst.

Fortunately, however, a method other than the alkali catalyst method can produce a fatty acid alkyl ester even with oil/fat having a high acid value. In the present embodiment, therefore, an esterification step is provided, which produces a fatty acid alkyl ester by a method other than the alkali catalyst method. This step may be referred to as a "second esterification step" in comparison with the previously described first separation step, which can be said as a first esterification step.

In the second esterification step, the first oil component separated in the first separation step may be used.

It is preferred to further use the second oil component separated in the above second separation step as a raw material. When the monohydric alcohol is recovered in the above alcohol separation step, it is also preferred to use the recovered monohydric alcohol as a raw material. The same raw materials (such as high acid value oil) as in the above first separation step (first esterification step) can also be used in combination as other raw materials.

By using these as the raw materials, it is possible to recycle industrial waste more efficiently in the production of a biodiesel fuel whose main components are hydrocarbons. Any method other than the alkali catalyst method can be preferably used even with these raw materials.

The method which can be employed in the second esterification step is a method other than the alkali catalyst method, and more specifically, an acid catalyst method, an acid-alkali catalyst method, a biocatalyst method, an ion-exchange resin method, a supercritical method, a subcritical method, and a solid catalyst method are exemplified. These methods can perform a transesterification reaction with a monohydric alcohol such as methanol even for waste cooking oil or oil/fat having a high acid value or for oil/fat that contains an unreacted free fatty acid.

In the second esterification step, glycerin is produced as a by-product together with the oil component which contains a fatty acid alkyl ester. The oil component obtained in the second esterification step and the glycerin liquid can be phase-separated by static placement, centrifugal separation, or the like. The separated oil component can be used as a biodiesel fuel or the like by recovering the fatty acid alkyl ester. On the other hand, the glycerin produced as a by-product can be supplied to the neutralization step, for example, together with the first glycerin liquid obtained in the above first separation step (first esterification step). With such a configuration, the glycerin produced as a by-product in the second esterification step can be used as a part of the glycerin liquid through the neutralization step, the second separation step, and the like, and can be further efficiently recycled.

It is particularly preferred to employ, as the second esterification step, the acid catalyst method among the above-described methods other than the alkali catalyst method.

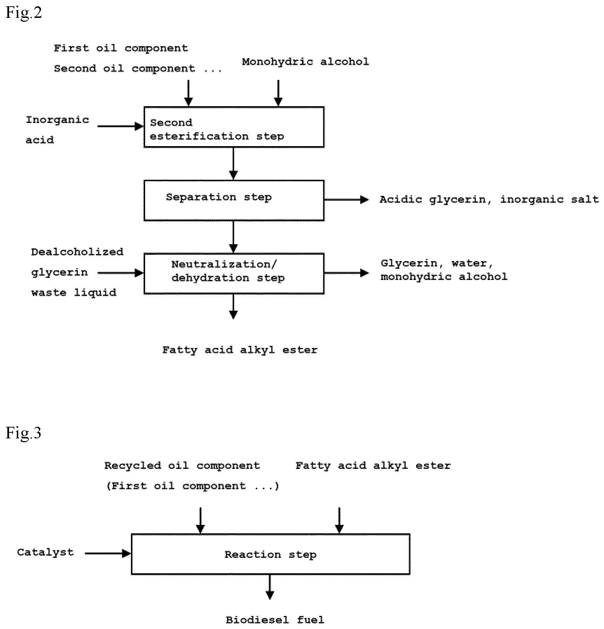

As illustrated in FIG. 2, when the acid catalyst method is employed as the second esterification step, the above first oil component and/or second oil component may be used as the raw materials. Other raw materials for use may include the monohydric alcohol recovered in the alcohol separation step and the same raw materials (such as a high acid value oil) as in the first separation step (first esterification step).

The reaction liquid obtained in the second esterification step may be separated into an oil component that contains a fatty acid alkyl ester and a glycerin liquid that contains glycerin produced as a by-product and an acid catalyst and its salt. Both the obtained oil component and the glycerin liquid are acidic, among which the acidic glycerin liquid can be supplied to the above neutralization step or the like.

On the other hand, it is preferred to perform neutralization, dehydration, or the like for the oil component which contains a fatty acid alkyl ester. Here, a method using waste glycerin produced as a by-product in the process of producing a biodiesel fuel is preferably exemplified as the method of neutralization/dehydration. Specifically, waste glycerin produced as a by-product in the process of producing the biodiesel fuel is dealcoholized and stored in a tank or the like, and the oil component to be neutralized is charged from the lower part of the tank to bring it into contact with the waste glycerin. This allows the acidic oil component to be neutralized by the alkali of waste glycerin, and the water and monohydric alcohol contained in the oil component are absorbed by the waste glycerin liquid. Then, the oil component charged from the lower part overflows from the upper part due to the difference in specific gravity and can therefore be easily recovered. With such a method, neutralization, dehydration, and dealcoholization can be performed at the same time, and a high-quality oil component can be easily obtained. The waste glycerin liquid which has absorbed water and monohydric alcohol can be supplied to the above-described neutralization step and can be made into a part of the second glycerin liquid through the second separation step and the like.

In the second esterification step, a biocatalyst method, a supercritical method, and a subcritical method can be preferably exemplified as methods other than the acid catalyst method.

The biocatalyst method is a method of promoting the transesterification reaction using lipase or phospholipase having the catalytic activity for the transesterification reaction. The biocatalyst method has a characteristic that the reaction conditions are moderate, but the transesterification reaction can be promoted even for oil/fat having a high acid value, and a by-product is less likely to be produced.

The supercritical method and the subcritical method are methods of promoting hydrolysis through adjusting the temperature and pressure to change the raw material to the supercritical state or subcritical state, thereby changing the phase state of a substance from two phases of gas and liquid to two phases of liquid and liquid, and further to one phase by lowering the dielectric constant, thus changing the reaction system, which originally requires the use of a catalyst, to a non-catalytic system.

By performing such a second esterification step, it becomes possible to recycle industrial waste further efficiently.

### <Catalytic Cracking Step>

This step is a step of bringing the first oil component obtained in the first embodiment or the fatty acid alkyl ester obtained in the second embodiment into contact with a catalyst to obtain a biodiesel fuel whose main components are hydrocarbons by a catalytic cracking method.

The catalyst used in this step is not particularly limited and can be used, provided that it is a catalytic cracking catalyst generally used in a catalytic cracking method for generating hydrocarbons. Examples thereof include oxides of alkaline earth metals, zeolite, sepiolite, alumina, silica, and clay minerals, and tungsten zirconia, FCC catalysts, etc. may also be used.

Among the above, magnesium oxide can be suitably exemplified as an oxide of an alkaline earth metal. High-purity magnesium oxide may be used as the magnesium oxide, and a magnesium oxide-containing composition such as blast furnace slag may also be used.

The catalyst used in this step is particularly suitable when it is supported on a porous carrier because in this case the contact area between the raw material and the catalyst is increased. Examples of the porous carrier include silica.

The usage ratio of the above catalyst to the above porous carrier is preferably 1:5 to 1:20, more preferably 1:7 to 1:15, and particularly preferably 1:9 to 1:10.

In the catalytic cracking step, it is preferred to feed and discharge an inert gas into and from of the reaction system as a carrier gas for recovering the obtained hydrocarbons. Nitrogen, argon, or the like can be used as the inert gas, and nitrogen is preferred. The flow rate of the carrier gas can be appropriately adjusted in accordance with the decomposition time of the oil component and fatty acid alkyl ester used as the raw materials. Specifically, as the flow rate of the carrier gas is increased, the reaction time is shortened and hydrocarbons having long carbon chains can be obtained. On the other hand, as the flow rate of the carrier gas is reduced, the reaction time is lengthened and hydrocarbons having short carbon chains can be obtained.

The catalytic cracking step preferably includes performing a reaction in a rotating drum that is arranged in a state in which its axis of rotation is inclined with respect to the horizontal plane.

A reactor used in the catalytic cracking method is generally of a type in which a reaction vessel is fixed and the catalytic cracking catalyst is agitated by a propeller or the like having a vertical axis of rotation. When such a reactor is used, however, there is a problem in that the catalytic cracking catalyst wears due to friction with the propeller or the like and the catalytic cracking catalyst is likely to be consumed.

On the other hand, by using as the reactor a rotating drum that is arranged in a state in which its axis of rotation is inclined with respect to the horizontal plane and performing the reaction in the drum, the catalytic cracking catalyst rolls with the rotation of the drum. This can sufficiently ensure the contact between the raw material (first oil or fatty acid alkyl ester) and the catalyst and suppress the consumption of the catalyst, and the catalyst replacement period can be extended.

When using the above rotating drum, the raw material and carrier gas are fed from one side of the drum along the axis of rotation of the drum. The hydrocarbons, which are reaction products, and the carrier gas are discharged from the other side of the drum (i.e., the opposite side to the feeding). By configuring the reactor in this manner, it is possible to feed the raw material and discharge the reaction products even while the drum is rotating, and this step can be carried out continuously.

In the catalytic cracking step, the temperature inside the reactor is adjusted so that the reaction temperature is 200°C to 600°C and preferably 300°C to 500°C. If the reaction temperature exceeds 430°C, the amount of light components increases, and the reaction temperature may therefore be adjusted from such a viewpoint. The reaction temperature can be confirmed by the temperature of the carrier gas discharged from the reactor.

The above reaction temperature is preferably adjusted by electromagnetic induction heating. Electromagnetic induction heating is excellent in terms of the energy efficiency.

In the catalytic cracking step, the catalyst is charged into the reactor, then the temperature inside the reactor is adjusted, and thereafter the raw material (including the first oil component and/or the fatty acid alkyl ester) is charged. Through this operation, the free fatty acid, which is contained in the first oil component, or the fatty acid alkyl ester is decomposed by the catalytic cracking method under the presence of the catalyst to generate hydrocarbons.

The generated hydrocarbons are discharged as gas from the reactor together with the carrier gas; therefore, they are cooled and condensed in a condenser or the like, and a composition that contains hydrocarbons is recovered as a liquid.

When it is attempted to obtain hydrocarbons having a specific chain length, the flow rate of the previously described carrier gas is appropriately adjusted, and the recovered hydrocarbon oil may be further distilled or fractionally distilled thereby to recover the hydrocarbons having a desired chain length.

The recovered composition contains hydrocarbons as the main components and can be used as a biodiesel fuel having good light oil quality.

According to the above method, a biodiesel fuel whose main components are hydrocarbons can be efficiently produced from a raw material that contains at least one of a free fatty acid and a fatty acid glycerin ester, especially from waste that contains a free fatty acid and/or a fatty acid glycerin ester. Therefore, wastes containing free fatty acids and/or fatty acid glycerin esters can be effectively recycled. Moreover, the glycerin moieties of the fatty acid glycerin ester can be separately recovered as glycerin by a transesterification reaction, and the glycerin moieties can therefore be effectively recycled without separately providing recovery facilities for lower hydrocarbons.

The first oil component obtained in the first embodiment undergoes the transesterification reaction once in the first separation step (first esterification step). On the other hand, the fatty acid alkyl ester obtained in the second embodiment undergoes the transesterification reaction twice in the first and second esterification steps. From the viewpoint of recycling the glycerin moieties of the fatty acid glycerin ester, it can be said that the second embodiment is more preferred. Moreover, in the second embodiment, the purity of the fatty acid alkyl ester is relatively high, and the properties of the raw material subjected to the catalytic cracking step are stable; therefore, the accumulation of ash in the catalytic cracking step is small, and even when the catalytic cracking catalyst is repeatedly used, reaction inhibition is less likely to occur. Thus, it can be said that the second embodiment is more preferred also from the viewpoint that the catalytic cracking step can be continuously performed for a long period of time.

The embodiments heretofore explained are described to facilitate understanding of the present invention and are not described to limit the present invention. It is therefore intended that the elements disclosed in the above embodiments include all design changes and equivalents to fall within the technical scope of the present invention.

### <Examples>

The present invention will be described below in more detail by illustrating examples and the like, but the present invention is not limited to the following examples and the like.

### (Preparation of Waste Glycerin)

Biodiesel fuel was produced through transesterification between waste cooking oil and methanol by an alkali catalyst method using potassium hydroxide as the catalyst. The by-product containing glycerin produced at that time was recovered as waste glycerin.

To this waste glycerin, 20 g of zeolite was added per 1 kg of the waste glycerin to remove water. The zeolite-added waste glycerin was passed through a 250 mesh filter to remove the zeolite and solid impurities.

The composition and physical properties of the waste glycerin as a raw material thus obtained (referred to as "raw material waste glycerin," hereinafter) are as listed in Table 1.

**[Table 1]**

| | Abstract | Unit | Value |
|---|---|---|---|
| Components | Glycerin | mass% | 45 |
| | Organic impurities | mass% | 43 |
| | Sodium hydroxide | mass% | 3.7 |
| | Water | mass% | 0.3 |
| | Methanol | mass% | 8 |
| Physical properties | Properties | - | Black viscous substance (water-soluble) |
| | pH | - | 10.7 |
| | Ash | mass% | 4.5 |
| | Total amount of heat generation | MJ/kg | 23 |

### (First Separation Step)

Into a reaction tank having a volume of 1000 L and having a heating/cooling function, 500 kg of the raw material waste glycerin and 300 kg of high acid value oil (150 mgKOH/g) were charged, and they were heated to 55°C while being stirred (120 rpm). In this state, 32 L of concentrated sulfuric acid was added into the reaction vessel over 15 minutes. When adding the concentrated sulfuric acid, attention was made so that the temperature of the mixture in the reaction vessel would not exceed 65°C. The pH of the reaction liquid after the total amount of concentrated sulfuric acid was added was 1. After the addition of concentrated sulfuric acid was completed, stirring was continued for 240 minutes. After that, the mixture was placed statically for 10 hours to separate it into an oil phase (first oil component) and an acidic glycerin phase (first glycerin liquid), and the first glycerin liquid (acidic glycerin phase, including precipitated potassium sulfate) was recovered. By repeating the above operation, 5000 kg of the first glycerin liquid was obtained.

### (Neutralization Step)

Into a reaction tank having a volume of 15000 L, 5000 kg of the first glycerin liquid and 5000 kg of waste glycerin were charged while being stirred. The pH was 5.0. After that, stirring was continued for 4 hours, and the mixture was then placed statically for 24 hours.

### (Second Separation Step)

The neutralized glycerin was treated with a decanter type centrifugal separator (product name: Z18H-V, available from TANABE WILLTEC INC.) at 5500 rpm for 180 minutes, and the precipitated potassium sulfate was separated and recovered. The liquid phase was further treated with a three-phase separation type centrifugal separator (available from Alfa Laval AB) at 8000 rpm for 180 minutes, and the second oil component, second glycerin liquid, and potassium sulfate were separated and recovered.

### (Alcohol Separation Step)

The second glycerin liquid obtained by three-phase separation was distilled to remove and recover methanol and water. The purity of the glycerin obtained by separating the methanol and water was 99 mass% (measured by gas chromatography).

### (Recovery of Recycled Oil Component)

After mixing the first oil component obtained in the first separation step and the second oil component obtained in the second separation step, the mixture was neutralized to about pH 6 using the waste glycerin, and the separated oil component was recovered. The recovered oil component was used as a "recycled oil component" and subjected to the catalytic cracking step to be described later.

### ((Second) Esterification Step)

The first oil component obtained in the first separation step and the second oil component obtained in the second separation step were mixed, to which the methanol recovered in the alcohol separation step was added, and concentrated sulfuric acid was added into the reaction vessel. Attention was paid that the temperature of the mixture in the reaction vessel would not exceed 65°C during the addition of concentrated sulfuric acid. The pH of the reaction liquid was 1 after the total amount of concentrated sulfuric acid was added. Stirring was continued for 240 minutes after the addition of concentrated sulfuric acid was completed. After that, the mixture was placed statically for 10 hours to separate into an oil phase and an acidic glycerin phase, and the oil phase was recovered. The obtained oil phase was a composition whose main component was fatty acid methyl ester (90 mass% or more). The obtained composition was neutralized and subjected to the catalytic cracking step to be described below.

### (Catalytic Cracking Step)

The recycled oil component obtained as above and the fatty acid methyl ester were used and subjected to a catalytic cracking method. Table 2 lists the properties of fatty acid methyl ester and recycled oil component used as the raw materials.

**[Table 2]**

| Evaluation item | Unit | Test method | Example 1 | Example 2 |
|---|---|---|---|---|
| | | JIS standard | Fatty acid methyl ester | Recycled oil component |
| Properties | | | Liquid, neutral | Liquid, neutral |
| Flash point | °C | K2265 | 174 | 258 |
| Pour point | °C | K2269 | -5.0 | -4.0 |
| Residual carbon content of 10% residual oil | mass% | K2270 | 0.07 | 0.13 |
| Kinetic viscosity (50°C) | mm²/s | K2283 | 4.236 | 1.921 |
| Sulfur content | mass% | K2541-1 | <0.0003 | <0.0003 |
| Density (15°C) | g/cm³ | K2249 | 0.8819 | 0.8922 |
| Ash | mass% | K2272 | 0.001 | 0.001 |
| Water | mass% | K2272 | 0.039 | 0.087 |
| Total amount of heat generation | MJ/kg | K2279-5 | 39.3 | 39.1 |

As the reactor in the catalytic cracking step, a rotating drum type reactor (capacity of 200 L) was used, having a rotating drum arranged in a state in which its axis of rotation was inclined at about 45° with respect to the horizontal plane.

The reactor was charged with 12.5 kg of silicon dioxide powder (CARiACT Q-3 available from FUJI SILYSIA CHEMICAL LTD., particle diameter: 1.18 to 2.36 mm) and 1.25 kg of magnesium oxide (available from Tateho Chemical Industries Co., Ltd., purity 99.99 mass%), and stirring in the reactor was started. The nitrogen flow rate was set to 2070 L/h, and the reactor temperature was maintained at 400°C by an electromagnetic induction heating device (EKOHEAT 20/10 available from Ameritherm).

After confirming that the discharged nitrogen temperature reached 380°C, fatty acid methyl ester was started to be charged into the reactor at a flow rate of 130 mL per minute.

After a while, the discharged gas was cooled by a condenser connected to the reactor outlet and began to accumulate as a liquid in a tank below the condenser, so the discharge valve was opened to collect the liquid in a sample bottle (Example 1).

In addition, a condensed liquid was obtained in the same manner as in Example 1, except that the recycled oil component was charged as substitute for the fatty acid methyl ester (Example 2).

The obtained liquid was subjected to gas chromatography (column used: DB1 available from AGILENT TECHNOLOGIES, INC., inner diameter=0.25 mm, length=30 m, film thickness=0.25 pm), and detection was performed with a hydrogen flame ionization detector (FID).

In both Example 1 using the fatty acid methyl ester as a raw material and Example 2 using the recycled oil as a raw material, a composition good in the light oil quality was confirmed, containing a hydrocarbon of pentadecane (C15) as the main component and further containing an alkane of C14, C13, C19, C17, and C16.

Specifically, in Example 1, C15 was 17.86% (peak area %, here and hereinafter), C14 was 9.02%, C13 was 6.78%, C16 was 4.60%, C17 was 3 .34%, C19 was 4.98%, and so on.

On the other hand, in Example 2, C15 was 23.62%, C14 was 7.45%, C13 was 3.50%, C17 was 4.01%, C20 was 8.37%, and so on.

In Example 1, the catalytic cracking catalyst maintained good catalytic activity even when the fatty acid methyl ester was charged at 2000 L/day and the operation was continued for 180 days or more. On the other hand, in Example 2, when the recycled oil component was charged at 2000 L/day, ash accumulated in the reactor, and deterioration of catalytic activity was recognized after about 10 days.

### [Industrial Applicability]

According to the present invention, it is possible not only to effectively recycle the industrial waste containing a free fatty acid and/or a fatty acid glycerin ester but also to produce a high value-added next-generation biodiesel fuel (biodiesel fuel whose main components are hydrocarbons), and the industrial utility value is high.

## Claims

1. A method for producing a biodiesel fuel, comprising:
a first separation step of mixing a raw material containing at least one of a free fatty acid and a fatty acid glycerin ester with an inorganic acid or enzyme to separate a first oil component and a first glycerin liquid;
an esterification step of reacting the first oil component with a monohydric alcohol to obtain a fatty acid alkyl ester by using a method other than an alkali catalyst method, the method being at least one method selected from a group consisting of an acid catalyst method, an acid-alkali catalyst method, a biocatalyst method, an ion-exchange resin method, a supercritical method, a subcritical method, and a solid catalyst method; and
a catalytic cracking step of bringing the fatty acid alkyl ester into contact with a catalyst to obtain a hydrocarbon.

2. The method according to claim 1, comprising:
a neutralization step of neutralizing, with an alkaline substance, the first glycerin liquid obtained by using the inorganic acid in the first separation step; and
a second separation step of separating a second oil component and a precipitated inorganic salt from the neutralized glycerin liquid,
wherein the esterification step includes reacting the second oil component together with the first oil component.

3. The method according to claim 1 or 2, comprising:
a neutralization step of neutralizing, with an alkaline substance, the first glycerin liquid obtained by using the inorganic acid in the first separation step;
a second separation step of separating a second oil component and a precipitated inorganic salt from the neutralized glycerin liquid; and
an alcohol separation step of separating a monohydric alcohol from the glycerin liquid from which the second oil component and the inorganic salt are separated,
wherein the monohydric alcohol separated in the alcohol separation step is used as the monohydric alcohol in the esterification step.

4. A method for producing a biodiesel fuel, comprising:
a first separation step of mixing a raw material containing at least one of a free fatty acid and a fatty acid glycerin ester with an inorganic acid or enzyme to separate a first oil component and a first glycerin liquid; and
a catalytic cracking step of bringing the first oil component into contact with a catalyst to obtain a hydrocarbon.

5. The method according to claim 4, comprising:
a neutralization step of neutralizing, with an alkaline substance, the first glycerin liquid obtained by using the inorganic acid in the first separation step; and
a second separation step of separating a second oil component and a precipitated inorganic salt from the neutralized glycerin liquid,
wherein the catalytic cracking step includes bringing the second oil component into contact with the catalyst together with the first oil component.

6. The method according to any one of claims 1 to 5, wherein the raw material in the first separation step contains at least one of a high acid value oil having an acid value of 10 mgKOH/g or more and waste glycerin produced as a by-product in a process of producing a fatty acid alkyl ester.

7. The method according to any one of claims 1 to 6, wherein the inorganic acid is used in the first separation step, and the inorganic acid is concentrated sulfuric acid.

8. The method according to any one of claims 1 to 7, wherein the inorganic acid is used in the first separation step, and a mixed liquid of the raw material and the inorganic acid has a pH of 3 or less.

9. The method according to any one of claims 1 to 8, wherein the catalyst in the catalytic cracking step contains magnesium oxide.

10. The method according to any one of claims 1 to 9, wherein the catalytic cracking step includes performing a reaction in a rotating drum that is arranged in a state in which its axis of rotation is inclined with respect to a horizontal plane.

11. The method according to any one of claims 1 to 10, wherein a reaction temperature in the catalytic cracking step is 200°C to 600°C.

12. The method according to claim 11, wherein the reaction temperature is adjusted by electromagnetic induction heating.
